Europäisches Patentamt

European Patent Office

Office européen des brevets

(11) Publication number: **0 232 706**
A2

# EUROPEAN PATENT APPLICATION

(21) Application number: **87100099.8**

(22) Date of filing: **07.01.87**

(51) Int. Cl.⁴: **G 01 N 33/80,** G 01 N 33/52, G 01 N 33/574 // G01N33/577

(30) Priority: **10.01.86 US 817874**

(43) Date of publication of application: **19.08.87** Bulletin 87/34

(84) Designated Contracting States: **DE FR GB**

(71) Applicant: **Sloan-Kettering Institute For Cancer Research, 1275 York Avenue, New York New York 10021 (US)**

(72) Inventor: **Cordon-Cardo, Carlos, 430 East 67th Street, New York New York 10021 (US)**
Inventor: **Lloyd, Kenneth O., 4525 Henry Hudson Parkway West, Bronx New York 10471 (US)**
Inventor: **Finstad, Connie L., 504 East 81st Street, New York New York 10028 (US)**
Inventor: **Mc Groarty, M.E., 16 Franklin Place, Great Neck New York 10021 (US)**
Inventor: **Bander, Neil H., 1385 York Avenue, New York New York 10021 (US)**
Inventor: **Old, Lloyd J., 600 West End Avenue, New York New York 10024 (US)**
Inventor: **Melamed, Myron R., 35 Braeside Lane, Dobbs Ferry New York 10522 (US)**

(74) Representative: **Patentanwälte Schulze Horn und Hoffmeister, Goldstrasse 36, D-4400 Münster (DE)**

(54) **Blood group antigen panel.**

(57) Monoclonal antibodies specific for human blood group antigens are useful in cancer diagnosis. It has been found that cancer patients often either express antigens of a blood type different from their normal blood type or cease to produce normal blood group antigens in cancerous tissues. By examining tissue samples with the monoclonal antibodies described herein, it becomes possible to diagnose cancer.

EP 0 232 706 A2

FIELD OF THE INVENTION

This invention relates to the use of monoclonal antibodies in determining the presence of particular antigens. Applications of this invention include, but are not limited to, diagnosis of disease, including cancer cell typing or classification and identification of precancerous lesions.

PRIOR ART

Blood group antigens are carbohydrate determinants which are typically found on erythrocytes, certain epithelial tissues, and in body secretions. They are formed by the sequential addition of saccharides to carbohydrate side chains of lipids and proteins. Hakomori, Seminars in Hematology 18:39 (1981). Genes control synthesis of these structures, as well as their expression in secretions, and on cell types other than erythrocytes (red blood cells).

The "A", "B" and "H" blood group antigens are known, at least indirectly, as identifying blood "type". Presence of "H" antigen only is characteristic of "Type O" blood, whereas presence of antigen "A" and "B" in the same sample is characteristic of type "AB". Presence of "A" or "B" antigen is characteristic of, respectively, type A or type B blood. Lewis antigens, i.e., Le$^a$ and Le$^b$, are typically found in plasma, secretions, and secretory epithelia.

- 2 -

These antigens are characteristic of other conditions, and determination of their presence is useful in areas other than blood typing. For example, Emmott, et al., J. Urol. 121:37 (1979) have found that the antigens of the ABH system which are usually present in normal urinary bladder tissue, are absent in urinary bladder tumors. Additional studies have shown that loss of these antigens is an early event in malignant transformation. Liss, et al., Am. J. Clin. Pathol. 68:372 (1977); (larynx carcinoma); Weinstein, et al., Cancer 43:661 (1979) (urinary bladder carcinoma). In patients with epithelial cancers, especially colon carcinomas, elevated levels of $Le^a$ and $Le^b$ antigens have been found. Koprowski, et al., Science 212:53 (1981). Additionally, the presence of normally incompatible blood groups in the same patient has been described in some cancer patients. Hatton, et al., Biochem. Biophys. Acta 666:361 (1981).

Determination of a change in the amount of blood group antigen, a sudden appearance, or disappearance, is indicative of a pathological state. Hatton, et al., supra, for example, found A antigen in type O cancer patients. Emmol, Lin, and Koprowski, supra have all shown that disappearance or appearance of antigens is typical of cancer. Hence, it is desirable to have a method for determining the presence of blood group antigens.

- 3 -

Several monoclonal antibodies, which are known to be specific for blood group antigens have been used to form a "panel" for determining particular antigens. H29-36 monoclonal antibody, for example, determines the presence of all varieties of A antigen. See, e.g., Sakamoto, et al., (unpublished manuscript), copending U.S. Patent Application Serial No. 474,415, "Monoclonal antibody S8 is known to detect B-antigen," Ueda, et al., PNAS 78:5122 (1981). Additionally, monoclonal antibodies T-174, T-218, P-12, and F-3 are specific for Le$^a$, Le$^b$, X, and Y antigens, respectively. Antibody K-21 detects precursor type antigen. Rettig, et al., Cancer Res. 45:815 (1985), Lloyd, et al., Immunogenetics 17:537 (1983).

These monoclonal antibodies all of which are described more fully, infra, are used as part of a panel to determine blood group antigens.

Further details on the panel, its uses, and further embodiments, are presented in the description which follows.

BRIEF DESCRIPTION OF THE FIGURES

Figure 1 shows pictorially the structures and origins of Le$^a$, Le$^b$, H-1, H-2, X, Y, A and B antigens.

- 4 -

Figures 2 and 3 illustrate immunohistological staining patterns of monoclonal antibodies described herein, when applied to normal human adult kidney and urothelium.

DETAILED DESCRIPTION OF PREFERRED EMBODIMENTS

Antibodies

The hybridoma cell lines which produce the monoclonal antibodies of this invention have been deposited with the American Type Culture Collection, 12301 Parklawn Drive, Rockville Maryland 20852 and bear the following accession numbers:

| Hybridoma | ATCC # |
|-----------|--------|
| H 29-36 | HB 8248 |
| S 8 | |
| T 174 | HB 8242 |
| T 218 | HB 8249 |
| P 12 | HB 8551 |
| F 3 | HB 8217 |
| K 21 | HB 8549 |

Information on derivation of these hybridomas may be found in copending U.S. Application Serial No. 474,415 (H 29-36, T-174, T-218), Serial No. 297,814 (S-8); Serial No.

- 5 -

604,080 (P-12) and K-21) and Serial No. 470,815 (F-3). In addition, the hybridomas are described in Ueda, et al., PNAS 78:5122 (1981) (S8); Rettig, et al., Cancer Res. 45:815 (1985) (P-12, and K-21) and Lloyd, et al., Immunogenetics 17:537 (1983) (F-3). The disclosures of all of these are incorporated by reference herein.

In summary, the hybridomas are prepared following the Kohler-Millstein method well known to the art, using, as immunizing cell lines, the materials set forth in Table 1.

Table 1. Derivation and specificity of mouse monoclonal antibodies identifying
Blood Group Antigens.

| Antibody (Ig subclass) | Immunizing Cell Line | Blood Group Specificity |
|---|---|---|
| K21 (μ) | Tera-1 Teratocarcinoma | Precursor (Type 1 Chain) |
| T174 (ɣ1) | SK-CO-10 Colon Cancer | Le$^a$ (Type 1 Chain) |
| T218 (μ) | SK-CO-10 Colon Cancer | Le$^b$ (Type 1 Chain) |
| P12 (μ) | Fresh Human Placenta | X (Type 2 Chain) |
| F3 (μ) | SK-LU-3 Lung Cancer | Y (Type 2 Chain) |
| T36 (ɣ3) | HT29 Colon Cancer | A (Type 1 and 2 Chains) |
| S8 (μ) | SK-RC-7 Renal Cancer | B (Type 2 Chain) |

Note: Purified agglutinin I from Ulex Europeans (Vector Laboratories, Burlingame, CA)
served to identify the H-antigen.

Because expression of ABO(H) antigens on the urogenital system has been discussed in the literature, e.g., Coon, et al., Am. J. Clin. Path. 76:163 (1981); Szulman, J. Exp. Med. 111:785 (1960), and recent studies have shown localization of $Le^a$ and $Le^b$ antigens in normal adult urothelium; Juhl, J. Histochem. Cytochem. 33:309 (1985), the urinary tract was used as an exemplary system. The following experiments provide an analysis which extends the study of the system to include Lewis, X, Y, and precursor determinants in the entire human urinary tract.

One skilled in the art will recognize that the analysis of human urinary tract tissue is applicable to any tissue system for the determination of expression of blood group antigens. For example, the terms "secretor" and "non-secretor" are used to define individuals who do or do not secrete A,B, or H antigens in saliva. "Secretors" produce $Le^b$ and Y antigens, whereas non-secretors produce $Le^a$ and X. Watkins, Science 152:172 (1966). As "secretor" or non-secretor" status, as well as changes in this status, is considered indicative of cancer susceptibility or onset, the monoclonal antibody panels, and methods described herein, are useful in cancer diagnosis.

MATERIAL AND METHODS

Tissues

Human fetal tissues ranging from 12 to 14 weeks of gestational age were obtained from elective abortions. Human normal adult tissues were obtained at autopsy within 9 hours post-mortem or from surgical pathology specimens within 1-2 hr of resection. Fresh tissues were fixed in 10% formaldehyde in phosphate buffered saline (PBS) (pH 7.4), and embedded in paraffin. Alternatively, tissues were snap-frozen in isopentane precoiled in liquid nitrogen, embedded in OCT compound in cryomolds and stored at -70°C until needed. Two fetal specimens containing kidney and ureter were studied, one expressing A group and the other H group antigens. The adult kidney, ureter, and/or bladder tissues chosen for the present study included samples from 3 group 0, 3 group A, 2 group B, and 2 group AB individuals. The blood group of individuals from whom the specimens were derived was correlated with the immunohistological patterns of reactivities.

Reagents.

Purified agglutinin I from Ulex europeus at 4 ug/ml served to identify the H-antigen. Mouse monoclonal antibody H 29-36 recognizes A antigen (all variants), mAb S8 detects B-antigen, mAbs T-174, T-218, P-12 and F-3 with

specificities for Le$^a$, Le$^b$, X and Y antigens, respectively, were also used. Finally, mAb K-21 detects precursor type I chain antigen. The antibodies were used as undiluted culture supernatants, or after purification from mouse ascities fluid (1:250 dilution).

Immunohistochemistry.

a) Indirect Immunofluorescence: Frozen tissues (4 to 8 microns) were cut using a cryostat with a microtome. Cryostat-cut sections were used unfixed or fixed for 10 minutes with either 1% formalin in PBS or cold acetone. Tissue sections were washed several times in PBS and rinsed in 2% bovine serum albumin in PBS (BSA-PBS). They were then incubated in a wet chamber with primary antibodies for 1 hour at room temperature, the titration and appropriate dilution having been previously established. Sections were washed with PBS and incubated for 45 minutes with secondary fluoresceinated antibodies, which have also been previously titrated for optimal dilution (usually 1:40 in BSA-PBS). Tissue sections were washed extensively in PBS with the creation of turbulence, using a magnetic stirring plate, wet mounted in 90% glycerol in PBS, and examined with a fluorescence microscope equiped with epifluorescence, using a 100 watt mercury lamp.

b)     Immunoperoxidase:  Formalin-fixed and paraffin-embedded sections were deparaffinized for this technique.  Sections were treated for 30 minutes in 1% hydrogen peroxide in PBS in order to remove endogenous peroxidase activity (no staining was observed when 1% periodic acid was used instead of 1% hydrogen peroxide). Tissue sections were washed several times in PBS, and then incubated with the appropriate supressor serum for 20 minutes.  Suppressor serum was drained off and sections were incubated with appropriately diluted primary antibody overnight at 4°C.  Both peroxidase-antiperoxidase and avidin-biotin methods were used in these experiment.  The secondary antibodies were horseradish peroxidase conjugated or biotinylated and they were incubated on sections for 1 hr.  Sections were then washed several times in PBS, and rinsed with 0.05M Tris buffer, 0.1 M NaCl, at pH 8.  For the final reaction diaminobenzidine (DAB) was used as chromogen, and the peroxidase reaction was performed by incubating tissue sections for 6 to 12 minutes with 5 mg of DAB tetrahydrochloride in 100 ml of tris buffer containing 100 ug of 0.3% hydrogen peroxide.  Sections were washed with distilled water, counterstained with hematoxylin, and mounted with permount.

Fresh frozen tissue sections were also used for this method. In this case, antibodies were incubated for 1 hr, and the other steps were similar to those described above for paraffin-embedded tissue sections.

c) Method for staining with lectin: The lectin Ulex europeus was incubated for 2 hours at room temperature, followed by rabbit anti-Ulex lectin antibody at a dilution of 1:1000 overnight at 4°C. Immunoperoxidase methods were performed as described above using biotinylated goat anti-rabbit immunoglobulins as secondary reagent.

d) Controls: Frozen and paraffin-embedded tissues expressing the appropriate blood group antigen served for titration of the reagents as well as positive and negative controls. Negative controls included substitution of the primary antibody by another antibody of the same species and isotype, or with PBS alone.

RESULTS

Table I summarizes the derivation of the panel of mouse mAb, their immunoglobulin subtype, and the characteristics of the blood group antigens detected. Table II summarizes the immunoreactivities of these antibodies on sections of normal adult kidney, ureter and urinary bladder.

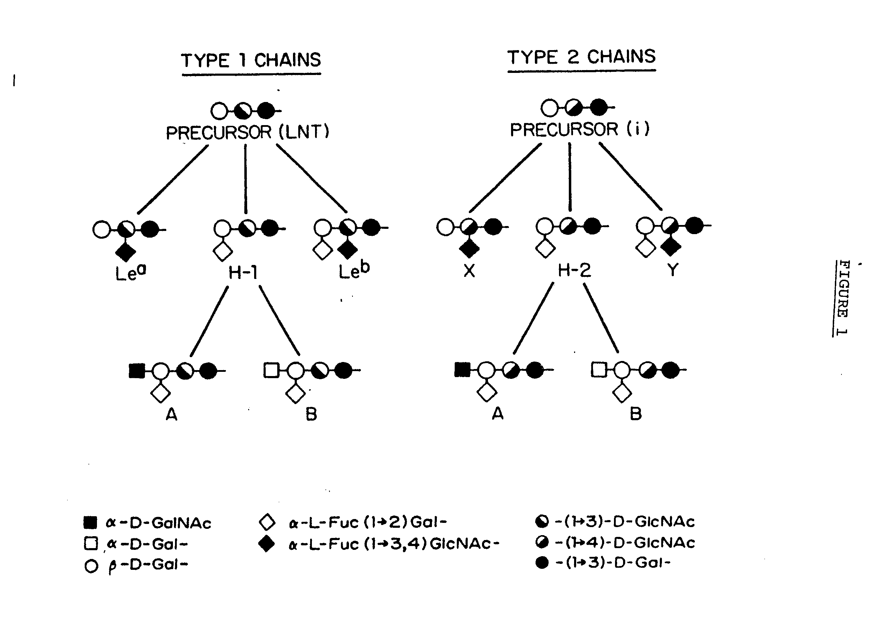

Table III summarizes the immunoreactivities of this panel of antibodies on sections of fetal kidney and ureter. Figures 1 and 2 illustrate the immunohistological staining patterns of these mAb with normal human adult kidney and urothelium.

### Blood group reactivities in adult tissues

Purified agglutinin I from Ulex europeus was used to identify the H-antigen. Expression of H-antigen was observed in endothelial cells and erythrocytes of all specimens studied. In the kidneys from A, B, or H individuals, H-antigen was found in the capillary network of glomeruli and also in the epithelial cells of collecting tubules with an homogeneous pattern of staining; the staining was weaker in the samples from AB individuals. All urothelial specimens expressed H-antigen throughout the mucosa, with an intense immunostaining of the basal layers. The rest of the nephron and connective tissue were negative for Ulex reactivity in all individuals tested.

Anti-A (H 29-36) and anti-B (S8) antibodies reacted only with the appropriately matched tissue specimens, that is from blood group A positive and B positive individuals, respectively. In each case, endothelial cells and erythrocytes were found to stain with the corresponding antibody. In the kidney, A and/or B antigens were found in glomerular and peritubular capillaries and epithelial cells of collecting tubules (Fig. 1A). Urothelium was immunoreactive throughout, with some variation in staining intensity and usually greater reactivity in luminal cells (Fig. 1B).

Lewis antigens were expressed on the nephron with distinct patterns of reactivities in the adult kidney. Lewis[a] (T174) was generally observed in the adult kidney in the epithelial cells of collecting and distal tubules (Fig. 2A), and in one case (AB specimen) a faint staining of the proximal tubules and portions of the Henle's loop was observed. In urothelium, Lewis[a] was found to be consitently positive in the superficial epithelial cell layers (Fig. 2B) and weak or absent in deeper cell layers, though in one sample (H specimen) there was positive staining through all layers of the epithelium. Lewis[b] (T218) was found in rare collecting ducts and sometimes single cells of the collecting duct of adult kidneys, but with intense reactivity (Fig. 2C). In urothelium, Lewis[b] was expressed mainly in the basal and suprabasal cell layers (Fig. 2D), or in some specimens throughout the entire epithelium with increased intensity in the basal cells. X determinant (P12) was detected in polymorphonuclear leukocytes. In adult kidney, X antigen was positive in epithelial cells of proximal tubules, portions of the loop of Henle (Fig. 2E), and in one case (A specimen) faint staining of distal and collecting tubular epithelial cells was observed. The reactivity of anti-X in urothelium was consistently intense in the umbrella cells, with only weak and variable staining of intermediate cell layers (Fig. 2F). Y determinant (F3) was detected in endothelial cells and erythrocytes. In adult kidney, endothelial cells of capillaries in the glomeruli were immunoreactive, as were the epithelial cells of collecting ducts (Fig. 2G). The reactivity with adult urothelium was intense and Y antigen was seen as an homogeneous pattern in the entire mucosa, with increased staining of basal and suprabasal cells (Fig. 2H).

Finally, the precursor type I chain (K21) was found in the adult kidney on occasional epithelial cells of the distal tubules and collecting ducts (Fig. 1C), and it was noted detected in any specimen of adult ureter or urinary bladder studied (Fig. ID).

Blood group reactivities in fetal tissue

No differences were observed in the immunostaining patterns of fetal tissues when compared with those of the adult with the reagents detecting H, A, and B antigens.

In general, the expression of Le$^a$, Le$^b$, X, Y and precursor antigens in the fetal urinary tract resembled that of the adult although there were some significant differences (Table II). Lewis$^a$ staining was very strong in the collecting ducts of the fetal kidney, while staining of urothelium was weaker in fetus than in adult. X antigen reactivity in the kidney was similar to the adult; the fetal urothelium was more strongly reactive than adult. Le$^b$ and Y antigen reactivities were very similar in adult and fetal tissues throughout the urinary system.

Finally, in contrast to adult tissues, fetal kidney and ureter expressed precursor type I antigen as strong and homogeneous staining of epithelial cells of collecting tubules and urothelium.

One skilled in the art will see the applicability of the monoclonal antibodies and panels of monoclonal antibodies described herein. For example, tissues and organs may be typed according to expression of blood group antigens to determine if they may be used in transplantation. The blood group antigen profile of the organ and/or tissue to be transplanted is compared to the blood group antigen profile of the intended recipient to determine if cross reactivity is to be expected. Blood typing can be done also, again using the antibodies and antibody panels described herein. A sample of blood is contacted to an antibody or panel of antibodies under conditions which favor complexing of the blood group antigens present with the known antibodies. By examining the pattern of complexing, one therefore determines the presence, or lack thereof, of particular blood group antigens. In typing organs and tissues, a similar practice is used (i.e., a cell sample or tissue sample is used for the contacting to the antibodies.

One skilled in the art will also see that in cancer patients, blood group antigen expression often changes, and different changes are characteristic of particular cancerous conditions. Thus, when cancer is suspected, a patient's blood, body secretions, or samples of tissue, are assayed using the monoclonal antibodies and antibody panels of this invention. Following the contacting method described supra, a pattern of blood group antigen expression is obtained, which is compared to an individual's normal blood group antigen expression panel, or "blood type." A diagnosis can then be made regarding the patient's condition with respect to cancer.

Hence, by using a panel of monoclonal antibodies including at least one antibody from the group consisting of H 29-36, S-8 T-174, T-218, P-12, F-3, and K-21, the aforementioned aspects of this invention are accomplished. As will be seen, _supra_, this invention is particularly useful in diagnosing cancer of the larynx, respiratory tract, and urinary bladder or urinary tract cancer.

One skilled in the art will see also that the antibodies and panel of antibodies described herein can be obtained in the form of kits, wherein different samples of monoclonal antibodies are separately packaged, such that individual antibodies, or the entire panel may be used, as desired. The individual samples allow one to perform sequential testing, for example.

While there have been described what are at present considered to be the preferred embodiments of this invention, it will be obvious to those skilled in the art that various changes and modifications may be made therein without departing from the invention, and it is, therefore, aimed to cover all such changes and modifications as fall within the true spirit and scope of the invention.

- 18 -

TABLE .11. IMMUNOREACTIVITY OF A PANEL OF ANTIBODIES DETECTING BLOOD
GROUP ANTIGENS IN ADULT KIDNEY, URETER AND URINARY BLADDER.

| BLOOD TYPE | TISSUES | ANTIGEN EXPRESSION | | | | | | | |
|---|---|---|---|---|---|---|---|---|---|
| | | H | A | B | LeA | LeB | X | Y | PS |
| H(0) | GLOMERULUS | 0 | 0 | 0 | 0 | 0 | 0 | 0 | 0 |
| | PROXIMAL TUB. | 0 | 0 | 0 | 0 | 0 | ● | 0 | 0 |
| | LOOP HENLE | 0 | 0 | 0 | ● | 0 | ● | 0 | 0 |
| | DISTAL TUB. | 0 | 0 | 0 | 0 | 0 | 0 | 0 | 0 |
| | COLLECTING TUB. | ● | 0 | 0 | ● | 0 | 0 | ● | ● |
| | UROTHELIUM | ● | 0 | 0 | ●/● | ●/● | ● | ● | 0 |
| A | GLOMERULUS | 0 | 0 | 0 | 0 | 0 | 0 | 0 | 0 |
| | PROXIMAL TUB. | 0 | 0 | 0 | 0 | 0 | ● | 0 | 0 |
| | LOOP HENLE | 0 | 0 | 0 | 0 | 0 | ● | 0 | 0 |
| | DISTAL TUB. | 0 | 0 | 0 | ● | 0 | ● | 0 | 0 |
| | COLLECTING TUB. | ● | ● | 0 | ● | ● | ● | ● | ●/● |
| | UROTHELIUM | ● | ● | 0 | ● | ● | ● | ● | 0 |
| B | GLOMERULUS | 0 | 0 | 0 | 0 | 0 | 0 | 0 | 0 |
| | PROXIMAL TUB. | 0 | 0 | 0 | 0 | 0 | ● | 0 | 0 |
| | LOOP HENLE | 0 | 0 | 0 | 0 | 0 | ● | 0 | 0 |
| | DISTAL TUB. | 0 | 0 | 0 | ● | 0 | 0 | 0 | ● |
| | COLLECTING TUB. | ● | 0 | ● | ● | ● | 0 | ● | ● |
| | UROTHELIUM | ● | 0 | ● | ● | ● | ● | ● | 0 |
| AB | GLOMERULUS | 0 | 0 | 0 | 0 | 0 | 0 | 0 | 0 |
| | PROXIMAL TUB. | 0 | 0 | 0 | ● | 0 | ● | 0 | 0 |
| | LOOP HENLE | 0 | 0 | 0 | ● | 0 | ● | 0 | 0 |
| | DISTAL TUB. | 0 | 0 | 0 | ● | 0 | 0 | 0 | ● |
| | COLLECTING TUB. | ● | ● | ● | ● | ● | ● | ● | ● |
| | UROTHELIUM | ● | ● | ● | ● | ● | ● | ● | 0 |

PS=PRECURSOR STRUCTURE ; IMMUNOREACTIVITIES: ●=HOMOGENEOUS STAINING,
●=OCCASIONAL CELLS, ●=LUMINAL SIDE POSITIVITY, ●=BASAL SIDE POSITIVITY.

TABLE III. IMMUNOREACTIVITIES OF A PANEL OF ANTIBODIES DETECTING BLOOD GROUP ANTIGENS IN FETAL KIDNEY AND URETER.

| TISSUES | ANTIGEN EXPRESSION | | | | | | | |
|---|---|---|---|---|---|---|---|---|
| | H | A | B | Le$^A$ | Le$^B$ | X | Y | PS |
| GLOMERULUS | 0 | 0 | 0 | 0 | 0 | 0 | 0 | 0 |
| PROXIMAL TUB. | 0 | 0 | 0 | 0 | 0 | ● | 0 | 0 |
| LOOP OF HENLE | 0 | 0 | 0 | 0 | 0 | θ | 0 | 0 |
| DISTAL TUB. | 0 | 0 | 0 | 0 | 0 | 0 | 0 | 0 |
| COLLECTING TUB. | ● | ● | ● | ● | θ | 0 | θ | ● |
| UROTHELIUM | ● | ● | ● | θ | θ | ● | ● | ● |

PS = PRECURSOR STRUCTURE; IMMUNOREACTIVITIES: ●=HOMOGENEOUS STAINING, θ=OCCASIONAL CELLS, θ=LUMINAL SIDE POSITIVITY, θ=BASAL SIDE POSITIVITY. TUB.=TUBULES.

What is Claimed:

1. Panel for identifying blood group antigens comprising at least one monoclonal antibody which specifically binds to a blood group antigen.

2. Panel of Claim 1, comprising at least one monoclonal antibody from the group consisting of H-29-36, S-8, T-174, T-218, P-12, F-3 and K-21.

3. Panel of claim 1, comprising monoclonal antibodies H-29-36, S-8, T-174, T-218, P-12, F-3, and K-21.

4. A method of typing blood comprising contacting a blood sample to a panel of antibodies which specifically bind to blood group antigens under conditions favoring formation of antibody-antigen complexes, and determining which antibodies specifically bind to antigens in said sample.

5. A method as in Claim 4, wherein said method is used to determine suitability of organ or tissue transplants.

6. A method of diagnosing disease associated with secretion of blood group antigens comprising contacting a tissue sample to a panel of monoclonal antibodies which specifically bind to different blood group antigens under conditions favoring formation of antibody-antigen complexes, determining presence of said complexes so as to determine presence of said antigens, and comparing antigen presence to antigen patterns associated with a particular disease.

7. Method of Claim 6, wherein said disease is cancer.

8. Method of Claim 7, wherein said cancer is larynx, respiratory tract, urinary bladder or urinary tract cancer.

9. A kit for use in determining the presence of blood group antigens comprising separate portion of at least one monoclonal antibody which specifically binds to a blood group antigen.

10. Kit of Claim 10, comprising at least one monoclonal antibody from the group consisting of H 29-36, S-8, T-174, T-218, P-12, F-3, and K-21.

TYPE 1 CHAINS

TYPE 2 CHAINS

PRECURSOR (LNT)

PRECURSOR (i)

Le$^a$

H-1

Le$^b$

X

H-2

Y

A

B

A

B

■ α-D-GalNAc
◇ α-L-Fuc (1→2) Gal-
◖ -(1→3)-D-GlcNAc

□ α-D-Gal-
◆ α-L-Fuc (1→3,4) GlcNAc-
◔ -(1→4)-D-GlcNAc

○ β-D-Gal-
● -(1→3)-D-Gal-

FIGURE 1

0232706